# FASCICULE DE BREVET EUROPEEN

(11) **EP 0 876 168 B1**
(45) Date de publication et mention de la délivrance du brevet: **31.07.2002**
(21) Numéro de dépôt: 96944103.9
(22) Date de dépôt: 30.12.1996
(51) Int. Cl.: A61M 1/10

(54) **POMPE D'ASSISTANCE CARDIAQUE IMPLANTABLE DU TYPE A BALLONNET DE CONTREPRESSION**
IMPLANTIERBARE HERZUNTERSTÜTZUNGSPUMPE MIT GEGENDRUCKBALLON
IMPLANTABLE HEART ASSISTANCE PUMP WITH A COUNTER-PRESSURE BALLOON

(30) Priorité: 26.01.1996 FR 9600949
(43) Date de publication de la demande: 11.11.1998
(73) Titulaire: Synthelabo Biomedical, 92350 Le Plessis-Robinson (FR)
(72) Inventeur: FRANCHI, Pierre, F-94400 Vitry-sur-Seine (FR)
(74) Mandataire: Dupuis-Latour, Dominique
(86) Numéro de dépôt international: FR9602104
(87) Numéro de publication internationale: WO9726929

(56) Documents cités:
- DE-A- 2 149 026
- DE-A- 2 619 239
- DE-U- 9 308 264
- GB-A- 1 265 655
- US-A- 3 007 416
- US-A- 4 250 872

## Description

L'invention concerne une pompe d'assistance cardiaque implantable du type à ballonnet de contrepression.

La technique du ballonnet intraaortique de contrepression est bien connue pour apporter une assistance hémodynamique efficace au ventricule gauche en cas d'insuffisance cardiaque congestive : le ballonnet, introduit dans la branche descendante de l'aorte, est gonflé pendant la phase diastolique du cycle cardiaque et injecte de ce fait un volume de sang supplémentaire dans le réseau artériel en amont et en aval de sa position. Dégonflé pendant la systole cardiaque consécutive, il diminue la charge du ventricule gauche et permet ainsi d'accroître le débit sanguin. Le bilan hémodynamique est positif : augmentation de la fraction d'éjection, diminution de la pression télédiastolique. Ainsi, le ballonnet délivre le complément d'énergie que le ventricule n'est plus en mesure de fournir, et l'état du patient est très sensiblement amélioré.

On a déjà proposé des systèmes implantés permettant de mettre en oeuvre cette technique de façon entièrement autonome, comme cela est par exemple décrit dans le US-A-5 222 980.

Ce document décrit une pompe d'assistance cardiaque implantable permanente insérée dans l'aorte descendante, fonctionnant sur le principe précité du ballonnet de contrepression, et constituée d'une membrane souple et élastique ayant la forme d'un manchon dont l'axe est confondu avec celui de l'aorte et disposé en lieu et place d'un tronçon d'aorte réséqué. La membrane est contenue dans une chambre rigide épousant sensiblement la forme de la membrane au repos, et dans laquelle l'injection d'un fluide hydraulique en provenance d'un générateur externe a pour effet de comprimer la membrane et donc de réduire le volume de sang qu'elle contient. À l'inverse, l'extraction du fluide provoque l'augmentation de son volume intérieur, donc le remplissage de la pompe.

L'un des problèmes rencontrés dans la conception de ce type d'appareils réside dans la fiabilité extrême qu'ils doivent présenter, du fait de leur caractère implanté. En effet, une durée de fonctionnement de cinq ans correspond à environ 300 000 000 de cycles que tous les éléments du système implanté doivent pouvoir subir sans dégradation ni défaillance.

À cet égard, le choix du matériau de la membrane souple et la définition de celle-ci sont des aspects critiques dans la conception d'une pompe d'assistance cardiaque implantable.

Outre la durée de vie compatible avec une implantation durable, il est indispensable que la pompe soit conçue de manière à minimiser les risques d'incidents thrombotiques, qui constituent l'une des difficultés majeures posées par les prothèses cardiovasculaires.

À cet effet, l'agencement de la pompe, notamment la définition de la membrane souple, doit répondre aux conditions suivantes :
― réduction des aspérités, turbulences, cavités et stases génératrices de thrombose ;
― élimination des écrasements et frictions aux zones de contact de la membrane sur elle-même ou sur les structures environnantes, générateurs d'hémolyse ;
― rinçage complet de l'espace intérieur de la membrane par le flux sanguin à chaque systole cardiaque ;
― fonctionnement en combinaison avec un traitement pharmacologique, local et continu, antagoniste des processus thrombotiques et expansifs, sans préjudice d'un traitement de la surface interne favorisant la colonisation par des cellules endothéliales autogènes.

L'un des buts de l'invention est de proposer une pompe d'assistance cardiaque implantable permettant de répondre à l'ensemble des contraintes et des conditions exposées ci-dessus, grâce à un agencement particulier des différents éléments de pompe, et tout particulièrement de la membrane souple.

Des modifications de la membrane ont été évoquées, par exemple dans le document DE-A-2 619 239.

La pompe est du type connu précité, c'est-à-dire comprenant : un corps essentiellement rigide ouvert à ses deux extrémités et destiné à être inséré dans l'artère aorte descendante ; une membrane souple et élastique en forme de manchon relié de manière étanche au corps en périphérie des extrémités de celui-ci de manière à définir, d'une part, entre corps et manchon, un espace intermédiaire fermé de volume variable et, d'autre part, à l'intérieur du manchon, un volume variable traversé par le sang à pomper ; et des moyens pour relier l'espace intermédiaire à une source de fluide hydraulique, propre à faire passer la membrane d'un état libre à un état tendu où elle se trouve sollicitée radialement vers l'intérieur, de manière à réduire corrélativement le volume traversé par le sang, et réciproquement.

Selon l'invention, entre les extrémités du corps la membrane est mobile librement par rapport au corps, et la membrane comporte au moins trois éléments raidisseurs longitudinaux, répartis régulièrement sur la périphérie de la membrane, ces éléments raidisseurs étant propres à diminuer localement l'élasticité longitudinale de la membrane en imposant à l'état tendu, en section droite, une forme étoilée se développant progressivement entre chaque région d'extrémité et la région médiane, cette forme étoilée laissant subsister un volume résiduel sans contact de la membrane sur elle-même.

Selon un certain nombre de caractéristiques avantageuses :
― les éléments raidisseurs longitudinaux sont dimensionnés localement de manière à limiter en tout point à une valeur limite prédéterminée le taux d'élongation du matériau de la membrane à l'état tendu et en fonction du profil extrême pris par la membrane dans cet état ; ces éléments raidisseurs longitudinaux sont de préférence des nervures formées sur la face externe de la membrane, dont la face interne comporte une concavité préformée au droit de ces nervures ;
― la membrane comporte en outre des éléments raidisseurs transversaux propres à diminuer localement l'élasticité transversale de la membrane.
― le corps comporte en outre au moins un élargissement annulaire périphérique en forme de canal annulaire ouvert en direction de la membrane et relié à au moins un conduit d'amenée du fluide hydraulique ;
― le corps peut être un cylindre de révolution droit, la membrane présentant à l'état libre une forme homologue de celle du corps, ou bien une forme de révolution à génératrice courbe cintrée extérieurement de manière à présenter dans la région médiane un diamètre supérieur à celui des régions d'extrémité ; dans ce dernier cas, la membrane peut présenter à l'état libre soit une forme de révolution homologue de celle du corps, soit une forme cylindrique droite ;
― il est prévu des moyens d'injection contrôlée d'un agent pharmaceutique antagoniste des thromboses et processus expansifs susceptibles de se développer localement ;
― le fluide hydraulique étant un fluide biocompatible et isotonique, il est prévu un réservoir accessible par voie percutanée ainsi que des moyens pour ajuster le volume du fluide et/ou sa salinité et pour éventuellement vidanger ce fluide et/ou le décharger dans le corps du patient si nécessaire.

On va maintenant décrire un exemple de réalisation de l'invention, en référence aux dessins annexés.

La figure 1 est une vue schématique montrant les différents organes constituant la pompe de l'invention.

La figure 2 est une demi-coupe longitudinale de l'ensemble corps et membrane, disposé à l'endroit du tronçon d'aorte réséqué.

La figure 3 est une coupe transversale, selon MM' de la figure 2, de cet ensemble dans une configuration où la membrane souple est à l'état de repos.

La figure 4 est une autre coupe transversale, selon OO' de la figure 2, de ce même ensemble.

La figure 5 montre la forme en section de la membrane souple en situation de déformation maximale, au voisinage de la région médiane.

La figure 6 est homologue de la figure 5, au voisinage d'une région d'extrémité.

La figure 7 représente deux demi-coupes longitudinales, selon YOZ de la figure 6, également pour une situation de déformation maximale de la membrane.

La figure 8 est un détail représentant des nervures transversales dans un plan axial.

La figure 9 est un détail représentant des nervures longitudinales.

La figure 10 illustre la manière dont la membrane souple se déforme progressivement au cours du gonflage.

Les figures 11 et 12 sont des coupes longitudinales schématiques illustrant deux variantes possibles de définition du corps et de la membrane de la pompe selon l'invention.

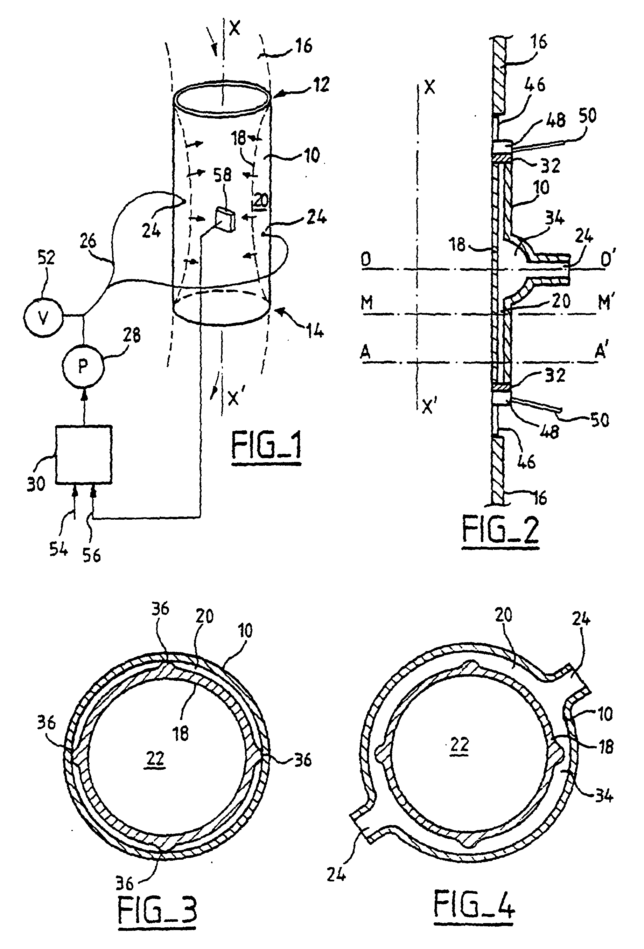

Sur la figure 1, on a représenté une pompe d'assistance cardiaque implantable, de type en elle-même connu, comprenant un corps rigide 10, typiquement en forme de cylindre de révolution (on verra toutefois plus loin que cette forme n'est pas limitative et que d'autres formes de révolution peuvent être envisagées). Ce corps est ouvert à ses deux extrémités 12 et 14 et inséré dans l'aorte descendante 16, l'axe de l'aorte et l'axe XX' du corps 10 étant confondus et ces deux éléments étant sensiblement de même diamètre.

Le corps rigide 10 contient une membrane souple 18, ayant au repos une forme homologue de celle du corps 10 de manière à épouser sensiblement la forme de ce dernier, auquel elle est solidarisée aux deux extrémités 12, 14 sur toute sa périphérie.

On définit ainsi entre le corps 10 et la membrane 18 un espace intermédiaire 20 fermé de volume variable et, à l'intérieur de la membrane 18, un espace central 22, également de volume variable, ce dernier volume diminuant lorsqu'augmente le volume 20, et inversement.

L'augmentation de volume de l'espace 20 résulte de l'injection d'un fluide hydraulique en un ou, de préférence, plusieurs points 24, ces points étant reliés par l'intermédiaire d'une conduite 26 à une source de pression variable 28 contrôlée par une électronique de commande 30. L'électronique 30 produit, en synchronisme avec les battements cardiaques, des variations de pression qui vont induire un gonflement ou un dégonflement du volume 20 et, corrélativement, une modification inverse du volume 22. Cette séquence de gonflements et dégonflements alternés produit un effet de pompage sur le volume de sang contenu en 22 et permet ainsi de soulager l'effort produit par le coeur (une systole du coeur correspondant à une diastole de la pompe d'assistance, et inversement).

Les figures 2 à 10 illustrent plus précisément la conception originale de l'ensemble corps et membrane selon l'invention.

La membrane 18 et le corps 10 sont solidaires l'un de l'autre en 32 à leurs extrémités amont et aval au niveau des secteurs annulaires de façon à réaliser l'étanchéité de l'espace vis-à-vis du conduit d'alimentation.

Le conduit d'alimentation 26 est abouché en 24 à un canal annulaire 34 (figures 2 et 4) pratiqué à la périphérie de l'enveloppe afin de faciliter l'écoulement et la répartition du fluide dans l'espace en vis-à-vis. Avantageusement, un conduit supplémentaire est abouché au canal en position diamétralement opposée au conduit, ou plusieurs conduits distribués régulièrement à la périphérie du canal, dans le but d'améliorer l'écoulement et la répartition du fluide et ainsi de neutraliser les forces de réaction engendrées par celui-ci et pouvant provoquer le déplacement latéral de la prothèse. La position du canal est de préférence médiane, comme illustré figure 2. D'autres canaux peuvent être disposés à différents niveaux sur la hauteur de la prothèse. La présence du canal annulaire 34 est importante pour minimiser les pertes de charge dans le circuit et obtenir un temps de réponse rapide et donc un contrôle précis du déplacement de la membrane en fonction du temps.

Le corps 10 est réalisé en une enveloppe rigide métallique, par exemple en titane, ou en une matière plastique biocompatible, cette enveloppe étant elle-même cylindrique.

Quant à la membrane 18, celle-ci est constituée d'un matériau élastomère tel qu'un silicone ou un polyuréthanne souple et élastique ; c'est une pièce ayant au repos une forme cylindrique telle qu'illustrée sur les figures 2 à 4.

Le choix du matériau et la définition de la membrane souple sont particulièrement critiques car, pour procurer une durée de vie suffisante (typiquement cinq ans), il faut absolument éviter que la membrane ne subisse en certains points des élongations trop importantes, à la longue génératrices de ruptures.

On connaît des élastomères de polyuréthanne qui peuvent travailler à des élongations pouvant aller jusqu'à 100 à 150 % sur plusieurs millions de cycles sans dégradations. Toutefois, pour être certain d'atteindre en toute sécurité un minimum de plusieurs centaines de millions de cycles sans dégradation, il importe de réduire l'élongation ponctuelle maximale du matériau à une valeur bien moindre, par exemple une déformation d'au plus 15 % en tout point de la membrane.

Une telle réduction de l'élongation en tout point procure une augmentation importante du nombre de cycles pouvant être atteints sans dégradation, et donc une durée de vie compatible avec celle d'un dispositif médical implanté.

Cette limitation en tout point de l'élongation maximale de la membrane sera obtenue de deux manières : d'une part par sa géométrie propre, que l'on va décrire, et d'autre part par l'ajout en tant que de besoin de nervures longitudinales, et éventuellement transversales, de manière à modeler l'élasticité à la fois longitudinale et transversale de la membrane de manière à respecter en tout point et en toute circonstances les contraintes imposées.

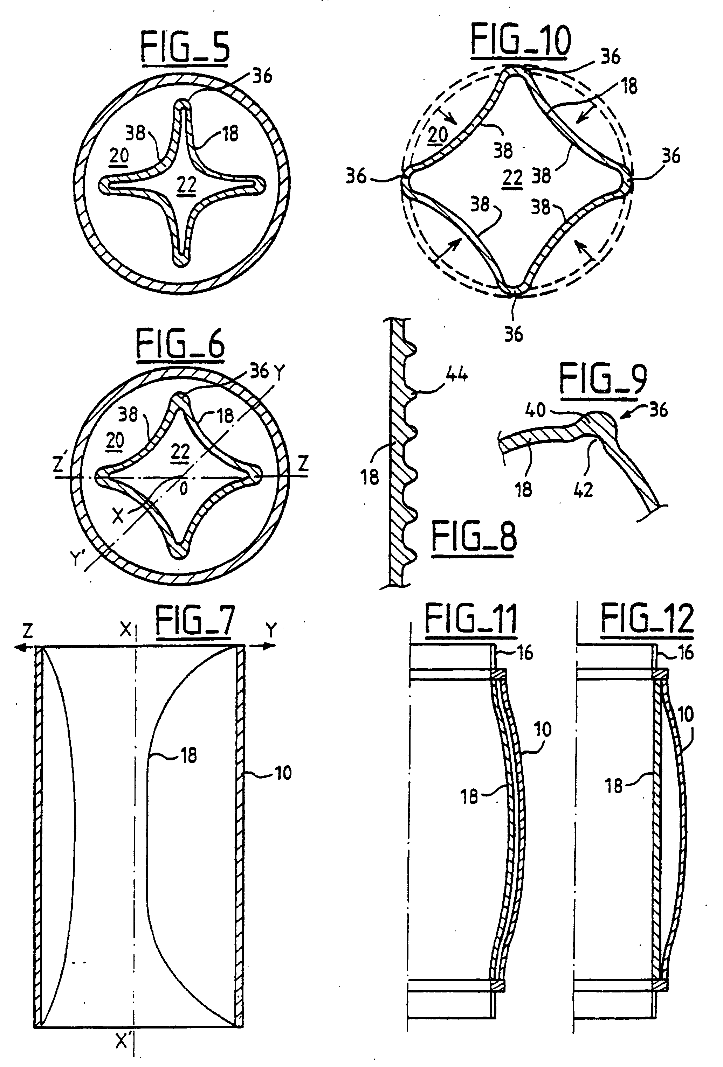

La forme de la membrane sur toute section droite est étoilée (figures 5 et 6) quelle que soit l'amplitude de la pression différentielle appliquée de l'extérieur vers l'intérieur sauf à l'endroit 32 de la fixation, en extrémité, où l'enveloppe impose une forme circulaire immuable. La forme étoilée se développe progressivement à partir de l'extrémité et atteint son maximum en position médiane.

La figure 7 représente deux demi-coupes dans un plan axial YY' et ZZ' dont la position angulaire est indiquées sur les figures 5 et 6, dans la même situation de pression différentielle maximale. Ainsi la demi-coupe YY' représente le profil de la membrane dans le plan axial médian entre deux nervures longitudinales, plan dans lequel la membrane possède le maximum de concavité et subit par conséquent l'élongation maximale. La demi-coupe ZZ' représente le profil de la membrane dans le plan axial passant par une nervure longitudinale ; sa concavité est moindre que la précédente du fait de sa plus faible élasticité.

La membrane comporte des nervures 36 disposées longitudinalement. Celles-ci sont régulièrement réparties à la périphérie, en nombre supérieur à deux, de préférence quatre. Elles constituent un renforcement de la membrane destiné à diminuer localement l'élasticité longitudinale. Elles ont pour effet de guider la déformation de la membrane lorsque celle-ci est soumise à une pression différentielle entre le sang à l'intérieur et le fluide hydraulique à l'extérieur, de manière à lui imposer une forme déterminée.

La figure 3 représente une coupe de la lumière de la membrane au voisinage de la région médiane (MM' sur la figure 2) en position d'ouverture maximale. Dans cette position la membrane et ses nervures ont un profil longitudinal rectiligne. Les figures 5 et 6 représentent respectivement la forme de la membrane sur deux sections, l'une au voisinage de la région médiane (MM' sur la figure 2), l'autre au voisinage de la région d'extrémité (AA' sur la figure 2), lorsque la pression différentielle appliquée entre l'extérieur et l'intérieur de la membrane est maximale et, par conséquent, le volume de sang contenu dans la prothèse est minimal.

L'élasticité longitudinale de la nervure et celle de la partie 38 de la membrane comprise entre deux nervures, qui sera appelée "paroi libre" peuvent être constantes sur toute la longueur ou être choisies indépendamment l'une de l'autre le long du profil longitudinal de façon à modeler la forme du profil longitudinal de la membrane et, par conséquent, moduler le volume minimum contenu dans la membrane et de l'optimiser en fonction de l'extension maximale autorisée par la fatigue de l'élastomère ― par exemple en limitant à 15 % l'élongation ponctuelle maximale.

Le modelage de l'élasticité permet aussi de réaliser une dissymétrie des profils de la membrane entre ses parties amont et aval dans le but de favoriser l'évacuation du sang vers l'amont ou l'aval, suivant la dissymétrie, lorsque la membrane est contractée.

On voit ainsi comment l'on peut, par une forme appropriée et la présence des nervures 36, modeler l'élasticité longitudinale de la membrane, élasticité longitudinale qui joue un rôle essentiel dans la détermination des profils longitudinaux et par conséquent des formes étoilées des sections transversales droites, en particulier dans le passage progressif de la forme circulaire imposée aux extrémités à la forme étoilée qui est celle de toute autre section droite de la membrane.

Cette configuration est en outre particulièrement avantageuse par le fait qu'elle génère peu de turbulences, tout particulièrement au moment de l'inversion de pression, du fait de la forme pré-définie de la membrane.

L'élasticité transversale de la paroi libre de la membrane est moins sollicitée. Il est néanmoins possible de moduler cette élasticité indépendamment de l'élasticité longitudinale au moyen de nervures transversales réparties régulièrement ou non le long du profil longitudinal, de raideur uniforme ou non.

La figure 8 représente des nervures transversales 44 dans un plan axial, apparaissant de préférence sur la face externe, la face interne demeurant lisse.

Les élasticités longitudinales (nervures et parois libres) et transversales (parois libres) constituent ainsi trois séries de paramètres indépendants permettant un réglage optimal de la déformation étoilée de la membrane, en particulier dans la recherche du volume résiduel minimal, sans provoquer de contact de la membrane sur elle-même et en maintenant une surface intérieure bien définie, lisse, sans discontinuité, propre à entretenir un écoulement sanguin sans turbulence. Si la membrane, tout en étant souple, n'était pas élastique, la forme étoilée ne pourrait s'établir de façon bien définie sur une large portion de la membrane à partir des extrémités, laissant celle-ci dans une situation de surface instable, sujette à des mouvements désordonnés générateurs de turbulences.

Les nervures de renforcement longitudinales et transversales sont de préférence réalisées par épaississement de la paroi de la membrane et obtenues directement au moulage de la pièce, la nervure étant de préférence située du côté externe. Néanmoins, les nervures longitudinales seront avantageusement préformées du côté interne comme indiqué en 40 sur la figure 9, qui représente une coupe transversale de la nervure ayant une concavité 42 sur la face interne destinée à réduire les tensions appliquées à la matière dans la zone de jonction entre paroi libre et nervure en position étoilée (figure 5).

La prothèse ainsi constituée est fixée à l'aorte en amont et en aval par l'intermédiaire de tronçons 46 (figure 2) de vaisseau artificiel de modèle courant, fixés aux extrémités de l'enveloppe rigide et suturés de façon classique sur l'artère.

À chacune des extrémités de l'enveloppe rigide, et solidaire de celle-ci, est disposé un anneau creux 48 contenant un produit pharmaceutique liquide alimenté de l'extérieur par l'intermédiaire de conduits 50. Cet anneau possède sur sa face intérieure, en communication avec le flux sanguin, des injecteurs régulièrement répartis à la périphérie ou bien un dispositif d'injection uniforme constitué par une membrane poreuse. L'injection est commandée de façon continue, séquentielle, programmée ou manuelle, et elle a pour but d'administrer un traitement local antagoniste des thromboses et processus expansifs pouvant se développer au contact de la zone de transition constituée par les sutures, des tronçons de vaisseau artificiel, de l'embouchure de la membrane et de la membrane elle-même.

L'injection sera avantageusement opérée par mise sous pression de la drogue de façon cyclique dans deux circonstances :
― au cours de la contraction diastolique, le flux sanguin chassé en amont et en aval de façon lente et laminaire se charge du produit pharmaceutique au passage de l'anneau et en balaye les zones sensibles périphériques immédiatement en aval du flux,
― au cours de la phase finale de la systole cardiaque, l'anneau situé en amont de la prothèse peut, inversement, diriger le flux chargé de produit pharmaceutique vers l'intérieur de la membrane.

Les injecteurs sont alimentés par un réservoir implanté (non illustré), maintenu sous pression, de façon continue ou de façon séquentielle au moyen d'électrovannes commandées par le système d'assistance cardiaque auquel appartient la pompe. La recharge du réservoir est effectuée par l'intermédiaire d'une chambre sous-cutanée à l'aide d'une aiguille hypodermique selon une technique connue.

Par ailleurs, le fluide hydraulique qui sert à exercer sur la membrane souple la contrepression recherchée est de préférence une solution saline aqueuse biocompatible, par exemple un sérum physiologique, de sorte qu'une fuite dans l'organisme ne soit pas toxique.

Le choix de ce fluide présente également une importance tenant au fait que, lorsque l'on utilise des polymères tels que ceux mentionnés plus haut (élastomères de polyuréthanne), ceux-ci présentent une osmose non négligeable, de l'ordre de 2000 à 2500 fois supérieure à celle d'une membrane en PTFE, de sorte qu'un certain passage de liquide et de gaz dissous peut avoir lieu au travers de cette membrane. Il est donc nécessaire de réduire au minimum le différentiel de pression osmotique.

La composition de ce fluide, plus particulièrement sa salinité, doit être ajustée de manière à obtenir l'équilibre des échanges croisés des composants au travers de la membrane en raison de sa perméabilité, ou que cet équilibre s'établisse spontanément par une variation du volume du fluide tolérable pour le générateur de flux hydraulique.

Le réservoir de fluide hydraulique comprendra avantageusement un septum 52 accessible par voie percutanée au moyen d'une aiguille hypodermique afin de permettre l'ajustement du volume de fluide et/ou sa salinité, ou de le vidanger.

Le séquencement de la pompe est contrôlé, comme on l'a indiqué plus haut, par l'électronique de commande 30 qui agit sur la source de pression 28. Cette électronique de commande reçoit d'une part des signaux de détection de l'activité cardiaque en 54. En complément, il peut être également prévu d'utiliser, en 56, des signaux provenant d'un capteur 58 de position de la membrane par rapport à l'enveloppe rigide dans le but de contrôler les mouvements de la membrane et, plus particulièrement, de permettre le réglage des positions extrêmes entre lesquelles elle doit se déplacer. Ce réglage est effectué par l'ajustement du volume du fluide en dégrossissage et, de façon plus fine, automatiquement par le générateur de fluide hydraulique.

Le capteur peut avantageusement être constitué d'un ou plusieurs couples formés d'un aimant permanent fixé à la membrane et d'un détecteur magnétique, de préférence un élément magnétorésistif, fixé sur l'enveloppe rigide en regard de son homologue.

En variante, si, pour quelque raison que ce soit, la position de la membrane devenait aberrante au risque de menacer l'écoulement du flux sanguin aortique, un dispositif de sécurité déclencherait automatiquement, ou sous commande manuelle, la décharge du fluide hydraulique dans le corps du patient, diminuant de ce fait la pression externe exercée sur la membrane et favorisant son retour sur une position de fonctionnement acceptable ou, à la limite, la plaçant en position de repos, neutralisée.

Le fonctionnement de la pompe est le suivant.

La membrane possède une élasticité sensiblement supérieure à celle de l'aorte native. À chaque instant la pression hydraulique appliquée à la face externe de la membrane est égale à la pression aortique appliquée à la face interne, augmentée de la composante due à la déformation élastique de la membrane, les forces d'inertie développées par la membrane étant négligeables par rapport à celles des fluides environnants. En conséquence, la membrane, partant d'une position de repos lorsqu'elle est en contact avec l'enveloppe rigide externe, se trouve soumise en fonctionnement à une pression différentielle dirigée de l'extérieur vers l'intérieur, aussi bien dans le mouvement de contraction que dans le mouvement inverse. La contraction est produite en début de diastole cardiaque, avec une durée comprise approximativement entre 300 et 600 ms, par injection de fluide hydraulique dans l'espace intermédiaire 20 entre membrane et enveloppe rigide.

Dans un premier temps, les nervures restent appliquées contre l'enveloppe rigide, tendues entre les deux fixations amont et aval, tandis que dans la partie médiane de la membrane (figure 10, section droite MM'), les secteurs cylindriques de celle-ci compris entre deux nervures (parois libres) inversent progressivement leur concavité, sans manifester de résistance élastique et, par conséquent, laissant la paroi prendre une forme indéterminée dans des limites étroites, jusqu'à atteindre la position opposée à la position initiale à partir de laquelle ils entrent en tension, prennent une forme déterminée et sollicitent les nervures qui se déplacent ensuite conformément à la représentation de la figure 5.

Le comportement au voisinage des extrémités est différent en raison de la fixation circulaire de la membrane. La forme de la membrane y reste essentiellement sous l'influence de l'élasticité longitudinale des parois libres et sous celle de la pression différentielle qui se développe dès que le processus d'inversion de concavité est achevé.

Le processus de retour au repos provoqué par le flux cardiaque systolique reproduit en sens inverse les mêmes phénomènes qu'à la contraction. Il dure de 150 à 250 ms.

Diverses variantes ou compléments peuvent être envisagés.

Ainsi, afin d'accroître la dynamique de la membrane, c'est-à-dire le volume de sang emmagasiné et rejeté au cours de chaque cycle, la membrane et l'enveloppe peuvent avantageusement prendre en position de volume maximal une forme de révolution à génératrice cintrée extérieurement (figure 11) dont la section droite médiane au repos de la membrane est un cercle de diamètre supérieur à celui des extrémités, donc de l'aorte.

En ce cas la membrane est une pièce montée naturellement cintrée à l'état libre. Le fonctionnement est semblable à celui qui a été décrit ci-dessus.

Selon une autre variante, illustrée figure 12, seule l'enveloppe rigide est cintrée, la membrane gardant la forme d'un cylindre droit au repos. Le fonctionnement est semblable à celui de la variante précédente, à la différence que la membrane atteint la position de volume maximal par application d'une pression différentielle inversée à partir de la position de repos. Cette disposition a pour effet de réduire la phase au cours de laquelle l'inversion de concavité de la paroi libre provoque une certaine instabilité de la surface.

## Revendications

1. Une pompe d'assistance cardiaque implantable du type à ballonnet de contrepression, comprenant :
― un corps (10) essentiellement rigide ouvert à ses deux extrémités (12, 14) et destiné à être inséré dans l'artère aorte descendante (16),
― une membrane souple et élastique (18) en forme de manchon relié de manière étanche au corps sur la périphérie des extrémités de celui-ci de manière à définir, d'une part, entre corps et manchon, un espace intermédiaire fermé (20) de volume variable et, d'autre part, à l'intérieur du manchon, un volume variable (22) traversé par le sang à pomper, et
― des moyens pour relier l'espace intermédiaire à une source de fluide hydraulique (28), propre à faire passer la membrane d'un état libre à un état tendu où elle se trouve sollicitée radialement vers l'intérieur, de manière à réduire corrélativement le volume traversé par le sang, et réciproquement,
pompe **caractérisée en ce que** :
― entre les extrémités du corps, la membrane est mobile librement par rapport au corps, et
― la membrane comporte au moins trois éléments raidisseurs longitudinaux (36), répartis régulièrement sur la périphérie de la membrane, ces éléments raidisseurs étant propres à diminuer localement l'élasticité longitudinale de la membrane en imposant à l'état tendu, en section droite, une forme étoilée se développant progressivement entre chaque région d'extrémité et la région médiane, cette forme étoilée laissant subsister un volume résiduel sans contact de la membrane sur elle-même.

2. La pompe de la revendication 1, dans laquelle les éléments raidisseurs longitudinaux (36) sont dimensionnés localement de manière à limiter en tout point à une valeur limite prédéterminée le taux d'élongation du matériau de la membrane à l'état tendu et en fonction du profil extrême pris par la membrane dans cet état.

3. La pompe de la revendication 1, dans laquelle la membrane comporte en outre des éléments raidisseurs transversaux (44) propres à diminuer localement l'élasticité transversale de la membrane.

4. La pompe de la revendication 1, dans laquelle les éléments raidisseurs longitudinaux (36) sont des nervures (40) formées sur la face externe de la membrane, dont la face interne comporte une concavité (42) préformée au droit de ces nervures.

5. La pompe de la revendication 1, dans laquelle le corps comporte en outre au moins un élargissement annulaire périphérique en forme de canal annulaire (34), ouvert en direction de la membrane et relié à au moins un conduit (24) d'amenée du fluide hydraulique.

6. La pompe de la revendication 1, dans laquelle le corps est un cylindre de révolution droit et la membrane présente à l'état libre une forme homologue de celle du corps.

7. La pompe de la revendication 1, dans laquelle le corps est une forme de révolution à génératrice courbe cintrée extérieurement de manière à présenter dans la région médiane un diamètre supérieur à celui des régions d'extrémité.

8. La pompe de la revendication 7, dans laquelle la membrane présente à l'état libre une forme de révolution homologue de celle du corps.

9. La pompe de la revendication 7, dans laquelle la membrane présente à l'état libre une forme cylindrique droite.

10. La pompe de la revendication 1, dans laquelle il est prévu en outre des moyens (48, 50) d'injection contrôlée d'un agent pharmaceutique antagoniste des thromboses et processus expansifs susceptibles de se développer localement.

11. La pompe de la revendication 1, dans laquelle le fluide hydraulique est un fluide biocompatible et isotonique, comprenant un réservoir (52) pour ce fluide accessible par voie percutanée ainsi que des moyens pour ajuster le volume du fluide et/ou sa salinité et pour éventuellement vidanger ce fluide et/ou le décharger dans le corps du patient si nécessaire.

## Patentansprüche

1. Eine implantierbare Herzhilfspumpe des Typs mit Gegendruck-Ballonett, aufweisend:
- einen im Wesentlichen steifen an seinen zwei Enden (12, 14) offenen Körper (10) und dazu bestimmt, in die hinuntergehende Aorta-Arterie (16) eingefügt zu sein,
- eine nachgiebige und elastische Membran (18) in Form einer Hülse, die in dichter Weise am Körper auf der Peripherie der Enden desselben in einer Weise verbunden ist, um einerseits zwischen Körper und Hülse einen geschlossenen Zwischenraum (20) mit variablem Volumen und andererseits im Inneren der Hülse ein variables Volumen (22) zu definieren, das durch das zu pumpende Blut durchquert wird, und
- Mittel, um den Zwischenraum mit einer Quelle hydraulischer Flüssigkeit (28) zu verbinden, die geeignet ist, die Membran von einem freien Zustand in einen gespannten Zustand, in dem sie radial in Richtung des Inneren belastet ist, übergehen zu lassen, in einer Weise, um korrelativ das durch das Blut durchquerte Volumen zu reduzieren und umgekehrt, wobei die Pumpe **dadurch gekennzeichnet ist, dass**:
- die Membran zwischen den Enden des Körpers im Verhältnis zum Körper frei bewegbar ist, und
- die Membran zumindest drei Längsversteifungselemente (36) aufweist, die gleichmäßig über die Peripherie der Membran verteilt sind, wobei diese Versteifungselemente geeignet sind, um lokal die Längselastizität der Membran zu verringern, wobei sie diese in den gespannten Zustand versetzen, im Querschnitt, wobei sich progressiv zwischen jedem Endbereich und dem mittleren Bereich eine Sternform entwickelt, wobei diese Sternform ein Restvolumen ohne Kontakt der Membran mit sich selbst bestehen bleiben lässt.

2. Pumpe nach Anspruch 1, bei welcher die Längsversteifungselemente (36) lokal derart dimensioniert sind, um an jedem Punkt den Entfemungsbetrag des Materials der Membran im gespannten Zustand auf einen vorbestimmten Grenzwert zu begrenzen und in Funktion des extremen Profils, das durch die Membran in diesem Zustand eingenommen wird.

3. Pumpe nach Anspruch 1, bei welcher die Membran unter anderem Querversteifungselemente (44) aufweist, die geeignet sind, die Querelastizität der Membran lokal zu verringern.

4. Pumpe nach Anspruch 1, bei welcher die Längsversteifungselemente (36) Rippen (40) sind, die auf der äußeren Fläche der Membran gebildet sind, deren innere Fläche eine Konkavität (42) aufweist, die geradlinig zu diesen Rippen vorgeformt ist.

5. Pumpe nach Anspruch 1, bei welcher der Körper unter anderem zumindest eine periphere kreisförmige Erweiterung in Form eines kreisförmigen Kanals (34) aufweist, der in Richtung der Membran offen ist und zumindest mit einer Leitung (24) für ein Zuführen der hydraulischen Flüssigkeit verbunden ist.

6. Pumpe nach Anspruch 1, bei welcher der Körper ein gerader Rotationszylinder ist und die Membran im freien Zustand eine Form aufweist, die homolog zu jener des Körpers ist.

7. Pumpe nach Anspruch 1, bei welcher der Körper eine Rotationsform ist mit außen einer Erzeugenden mit bogenförmiger Krümmung, um in dem mittleren Bereich einen Durchmesser aufzuweisen, der größer ist als jener der Endbereiche.

8. Pumpe nach Anspruch 7, bei welcher die Membran im freien Zustand eine homologe Rotationsform derjenigen des Körpers aufweist.

9. Pumpe nach Anspruch 7, bei welcher die Membran im freien Zustand eine gerade Zylinderform aufweist.

10. Pumpe nach Anspruch 1, bei welcher unter anderem Mittel (48, 50) für eine kontrollierte Injektion eines pharmazeutischen gegen Thrombosen wirkenden Mittels und expansive Prozesse, die geeignet sind, sich lokal zu entwickeln, vorgesehen sind.

11. Pumpe nach Anspruch 1, bei welcher das hydraulische Fluid ein biokompatibles und isotonisches Fluid ist, wobei sie ein Reservoir (52) für dieses Fluid aufweist, das zugänglich ist durch eine perkutane Bahn, sowie Mittel, um das Volumen des Fluids und/oder seinen Salzgehalt einzustellen und um gegebenenfalls dieses Fluid zu entleeren und/oder um es in den Körper des Patienten, falls dies notwendig ist, abzulassen.

## Claims

1. An implantable heart-assist pump of the back-pressure balloon type, the pump comprising:
― an essentially rigid body (10) open at both ends (12, 14) and designed to be inserted in the down aorta artery (16);
― a flexible and elastic membrane (18) in the form of a sleeve connected in sealed manner to the body on the periphery of the ends thereof so as to define firstly, between the body and the sleeve, a closed intermediate space (20) of variable volume, and secondly, inside the sleeve, a variable volume (22) through which the blood to be pumped passes; and
― means for connecting the intermediate space to a source of hydraulic fluid (28) suitable for causing the membrane to pass from a free state to a tensioned state in which it is urged radially inwards, thereby correspondingly reducing the volume through which the blood passes, and vice versa;
the pump being **characterized in that**:
― between the ends of the body, the membrane is freely movable relative to the body; and
― the membrane has at least three longitudinal stiffener elements (36) uniformly distributed around the periphery of the membrane, said stiffener elements being suitable for locally reducing the longitudinal elasticity of the membrane, imposing a star-shape to the right section of the membrane in the tensioned state, which shape develops progressively between each end region and the middle region, said star-shape leaving a residual volume without the membrane coming into contact against itself.

2. The pump of claim 1, in which the longitudinal stiffener elements (36) are locally dimensioned so as to ensure that the elongation percentage of the membrane material in the tensioned state is limited at all points to a predetermined limit value as a function of the extreme profile taken up by the membrane in said state.

3. The pump of claim 1, in which the membrane further includes transverse stiffener elements (44) suitable for locally reducing the transverse elasticity of the membrane.

4. The pump of claim 1, in which the longitudinal stiffener elements (36) are ribs (40) formed on the outside space of the membrane, with the inside face having a preformed concave shape (42) in register with the ribs.

5. The pump of claim 1, in which the body further includes at least one peripheral annular enlargement in the form of an annular channel (34) open towards the membrane and connected to at least one duct (24) for delivering hydraulic fluid.

6. The pump of claim 1, in which the body is a right circular cylinder and the membrane in the free state is similar in shape to the body.

7. The pump of claim 1, in which the body is circularly symmetrical, having an outwardly curved generator line so that its middle portion has a diameter greater than the diameter of its end portions.

8. The pump of claim 7, in which the membrane in the free state has a circularly symmetrical shape corresponding to that of the body.

9. The pump of claim 7, in which the membrane in the free state is in the form of a right cylinder.

10. The pump of claim 1, in which means (48, 50) are also provided for controlled injection of a pharmaceutical agent for opposing thromboses and expansive processes that may occur locally.

11. The pump of claim 1, wherein the hydraulic fluid is a biocompatible and isotonic fluid, including for said fluid a percutaneously-accessible container (52) provided together with means for adjusting the volume of the fluid and/or its salinity and optionally for emptying said fluid and/or discharging it into the body of the patient, should that be necessary.
